# EUROPEAN PATENT SPECIFICATION

(11) **EP 3 058 958 B1**
(45) Date of publication and mention of the grant of the patent: **19.09.2018**
(21) Application number: 16156402.6
(22) Date of filing: 18.02.2016
(51) Int. Cl.: A61K 9/14, A61K 9/19, A61K 38/12

(54) **ANIDULAFUNGIN COMPOSITION**
ANIDULAFUNGINZUSAMMENSETZUNG
COMPOSITION D'ANIDULAFUNGINE

(30) Priority: 23.02.2015 DE 102015203222
(43) Date of publication of application: 24.08.2016
(73) Proprietor: Selectchemie AG, 8038 Zürich (CH)
(72) Inventor: Doser, Karlheinz, 8810 Horgen (CH); van Boven, Marinus, 8802 Kilchberg (CH); Yuan, Jiandong, 215001 Suzhou Jiangsu (CN)
(74) Representative: Uexküll & Stolberg

(56) References cited:
- EP-A2- 1 582 204
- WO-A1-2009/076620
- WO-A1-2013/142279
- WO-A2-03/105767
- US-A1- 2009 286 764

## Description

### Field of the invention:

The present invention relates to a production method for compositions comprising anidulafungin as active ingredient being useful for the prevention and/or treatment of fungal infections. Said compositions additionally comprise specific bulking agents, stabilizers and surfactants and a buffering agent.

### Background of the invention:

Echinocandins are large lipopeptide molecules that are inhibitors of β-(1,3)-glucan synthesis, an action that damages fungal cell walls. The first Echinocandin to be approved was caspofungin, followed by micafungin and anidulafungin. Due to the poor absorption after oral administration the use is limited to the intravenous route. The 3 commercially available echinocandins are unstable in the solid state and must be stored under moderate refrigeration (micafungin sodium) or even at temperatures as low as -70°C (caspofungin acetate). Generally they are also unstable to light, humidity, acids and the like. Therefore the development of pharmaceutical preparations in which these peptide compounds and their salts are stabilized was necessary and for above the mentioned echinocandins lyophilized presentations were developed.

Caspofungin is marketed as a lyophilized powder and the excipients include sucrose, mannitol, acetic acid and sodium hydroxide (as described in WO 97/39763) and Micafungin powder contains as excipients lactose, citric acid and sodium hydroxide (as described in WO 2001/002002). Whereas caspofungin acetate and micafungin sodium are freely soluble in water, anidulafungin is not and for that reason, a part of a bulking agent (mannitol), a stabilizer (fructose as the originally developed active ingredient is a complex with fructose), a buffer and pH adjusting agents (tartaric acid, sodium hydroxide and hydrochloric acid), the anidulafungin powder also contains a commonly used nonionic surfactant sorbitan derivative as solubilizing agent (polysorbate 80). This formulation is described in EP 1 582 204.

For medical use excipients are preferred which are described in an Official Monograph e.g. the USP. Tartaric acid is not monographed in the USP for the use in parenteral preparations. For the preparation of infusion and injection solution other acids like lactic acid and phosphoric acids and their salts are preferred as buffer substances (e.g. lactated Ringer solution, USP 37, p 4587 and sodium phosphate injection, USP 37, p 4725).

Therefore, there is still a need for improved preparation methods for parenteral formulations with anidulafungin.

### Summary

Provided is here a stabilized pharmaceutical composition comprising:
a) a pharmaceutical effective amount of the active ingredient anidulafungin
b) a solubilizing agent
c) a stabilizing agent
d) a bulking compound
e) a buffer
f) a pH adjusting agent

The present inventors have surprisingly found during their formulation development that anidulafungin compositions with certain buffers different from tartaric acid are stable, and exhibit a minor formation of degradation products during storage under moderate refrigeration, at room temperature and at 40°C.

The formulation is prepared as a solution of 10 mg/ml of anidulafungin, 10 mg/ml of fructose, 50 mg/ml of mannitol, 25 mg/ml of polysorbate 80, 7.5 mmol/L of lactic acid, which is adjusted to pH 3.0 - 6.0, preferably 4.0 - 5.0 with sodium hydroxide.

The formulation is prepared as a solution of 10 mg/ml of anidulafungin, 10 mg/ml of fructose, 50mg/ml of mannitol, 25 mg/ml of polysorbate 80, 7.5 mmol/L of phosphoric acid, which is adjusted to pH 3.0 - 6.0, preferably 4.0 - 5.0 with sodium hydroxide.

### Detailed description:

The production and purification of the cyclic peptide anidulafungin and its carbohydrate complex (with fructose) has been described in WO03/105767 A2, EP 0 561 639, EP 1 137 663, EP 2 076 528 and EP 1 157 030 (carbohydrate complex).

From prior art, also for peptides and proteins, the preferred bulking agent to form a lyophilized cake is mannitol, and the preferred stabilizing agent is fructose (stabilizing agent used in the active ingredient, see EP 1 157 030).

The active ingredient and the stabilizing agent can be used in separate form. However, it is also possible to use these ingredients in a premixed form, e.g. in the form of a anidulafungin/carbohydrate complex. Preferably the complex of anidulafungin and fructose is used.

Suitable buffer agents (such as acetates, lactates, citrates, phosphates etc) and pH adjusting agents (sodium hydroxide, hydrochloric acid, etc.) are listed in PDA Journal of Pharmaceutical Science & technology, Vol.51, No.4/July-August 1997, pg166-171. The pharmaceutical formulations of the present invention do preferably not contain tartaric acid.

The solubilizing agent is preferably a sorbitan polyoxyethylened derivative (polysorbate), more preferably polysorbate 80 or polysorbate 20. Polysorbates are a class of nonionic surfactants. They are ethoxylated fatty acid esters of sorbitan. Polysorbate 80 is polyoxyethylene (20) sorbitan monooleate, and polysorbate 20 is polyoxyethylene (20) sorbitan monolaurate. The number 20 following the 'polyoxyethylene' part refers to the total number of oxyethylene -(CH₂CH₂O)- groups found in the molecule.

The present invention solely concerns a process, as defined by the appended claims, for making a freeze-dried pharmaceutical formulation comprising the steps of (i) preparing an aqueous solution comprising anidulafungin, a solubilizing agent, preferably polysorbate, a stabilizing agent, preferably fructose, a bulking agent, preferably mannitol, and a buffer, and (ii) freeze-drying the solution of step (i), wherein the anidulafungin used in step (i) is preferably used in the form of a powder which does not contain particles having a size of more than 400 µm.

Preferably a process comprising the following steps:
(a) dissolving the buffer in water for injection and adjust the pH with sodium hydroxide to 3.0 - 6.0, preferably 4.0 - 5.0;
(b) adding the solubilizing agent, the anidulafungin, the stabilizing agent, the bulking agent and the remaining water for injection, and stirring the solution;
(c) sterile filtering the solution of step (b);
(d) freeze-drying the solution of step (c).

In step (b) the various compounds are preferably added in the order specified above. Preferably the solubilizing agent is first dissolved in water for injection (WFI) and this solution is then added to the buffer of step (a).

Next the active ingredient is added. The inventors found that anidulafungin particles with a particle size of more than 400 µm dissolve only slowly in the solution of step (a) although a solubilizing agent is added. Even in small scale experiments the dissolution needs more than 4 hours. The dissolution can be accelerated by a pre-treatment of the active ingredient, preferably by dispersing the active ingredient in a pharmaceutically acceptable solvent, preferably in WFI, e.g. in an ultrasonic bath, and using this dispersion in step (b).

Nevertheless, this process is still not optimal for commercial production. It was surprisingly found that the dissolution of anidulafungin can be significantly accelerated by using anidulafungin in the form of a powder which does not contain particles with a diameter of more than 400 pm, preferably not more than 350 µm. Larger particles are preferably removed by sieving. This powder can be directly added is step (b). Preferably this powder is also used in the form of a dispersion as described above.

The mixture comprising the solubilizing agent and the active ingredient is preferably agitated until all components have dissolved completely. Then the stabilizing agent and the bulking agent are added and the mixture is preferably again agitated until complete dissolution has been achieved. Water can be added as necessary in order to achieve the desired final concentration of ingredients, and sodium hydroxide, preferably a solution of sodium hydroxide, or HCl solution may be added as required in order to adjust the pH to be within the above range. The solution is then sterilized, preferably by sterile filtration, and lyophilized. Agitation is preferably achieved by stirring, more preferably by stirring with a mechanical stirrer.

The process of the present invention wherein anidulafungin with a particles size of 400 µm or less, preferably of 350 µm or less is used is particularly suitable for large scale manufacturing of anidulafungin formulations.

In previous art, EP 1 582 204, formulations with acetic acid, citric acid and tartaric acid are described and their stability at 40°C is monitored during 4 weeks, measuring the increase of the total percentage of related substances. From the results of that stability study the obvious conclusion is that the formulation with tartaric acid as buffer was superior, meaning more stable, to other formulations with citric acid or acetic acid as buffer.

### Examples:

### Example 1

The pharmaceutical formulations were prepared by the following method: Dissolve the buffer with water for injection (70% of the total amount) and after adjusting the pH with 1M sodium hydroxide to pH 4.3, add and stir to dissolve the polysorbate 80 and cool down to 25°C. Add anidulafungin and stir to dissolve completely. Afterwards add fructose and mannitol and stir to dissolve and add the remaining water for injection. After filtration 5ml is transferred to a vial and then freeze dried in a lyophilizer.

The following pharmaceutical formulation examples were prepared in accordance with the previously described method. In Table 1 all weights are in milligrams and concentration in mg/ml.

**Table 1**

| **Pharmaceutical formulations of anidulafungin for injection** | | | |
|---|---|---|---|
| **Components** | **Composition** | | |
| | **Solutions** | **Formulation 1** | **Formulation 2** |
| Anidulafungin | 10 mg/ml | 50mg | 50mg |
| Mannitol | 50 mg/ml | 250mg | 250mg |
| Fructose | 10 mg/ml | 50mg | 50mg |
| Polysorbate 80 | 25 mg/ml | 125mg | 125mg |
| Lactic Acid | | 7.5 mmol/L | |
| Phosphoric Acid | | | 7.5 mmol/L |
| Sodium Hydroxide | suitable | suitable | suitable |
| Water for Injection | | Add to 5.0ml | Add to 5.0ml |

7.5 mmol/L: The concentration of liquid buffer salt before lyophilization.

The stability of the freeze-dried formulations was evaluated by monitoring a sample for the percent increase in the individual related substances by HPLC at initial, 5days-10days-3.5 months at 2-8°C, at 25°C and 40°C.

**Table 2**

| **Stability of the freeze dried anidulafungin pharmaceutical formulation with Lactic Acid as buffer** | | | | | | | | | |
|---|---|---|---|---|---|---|---|---|---|
| **Formulation 1 (Lactic Acid)** | RRT | 0.68 | 0.79 | 0.85 | 0.88 | 0.91 | 1 | 1.08 | 1.14 |
| 0 day | Area% | 0.01 | 0.01 | / | 0.02 | 0.07 | 99.76 | 0.08 | 0.03 |
| | | | | | | | | | |
| 2-8°C 5 days | Area% | 0.05 | / | / | 0.02 | 0.07 | 99.8 | 0.07 | / |
| 2-8°C 10 days | Area% | 0.05 | / | / | 0.02 | 0.07 | 99.76 | 0.06 | 0.02 |
| 2-8°C 3.5 months | Area% | 0.05 | / | / | 0.03 | 0.07 | 99.77 | 0.06 | 0.02 |
| | | | | | | | | | |
| 25°C 5 days | Area% | 0.07 | / | / | 0.02 | 0.07 | 99.75 | 0.07 | 0.01 |
| 25°C 10 days | Area% | 0.08 | / | / | 0.02 | 0.07 | 99.75 | 0.07 | 0.02 |
| 25°C 3.5 months | Area% | 0.11 | 0.01 | 0.01 | 0.03 | 0.06 | 99. 68 | 0.05 | 0.03 |
| | | | | | | | | | |
| 40°C 5 days | Area% | 0.20 | 0.02 | 0.01 | 0.01 | 0.06 | 99.60 | 0.05 | 0.03 |
| 40°C 10 days | Area% | 0.24 | 0.02 | 0.02 | 0.02 | 0.07 | 99.53 | 0.04 | 0.04 |
| 40°C 3.5 months | Area% | 0.58 | / | 0.07 | 0.10 | 0.05 | 98.39 | 0.03 | 0.55 |

**Table 3**

| **Stability of the freeze dried anidulafungin pharmaceutical formulation with Phosphoric Acid as buffer** | | | | | | | | | |
|---|---|---|---|---|---|---|---|---|---|
| **Formulation 2 (Phosphoric Acid)** | RRT | 0.68 | 0.79 | 0.85 | 0.88 | 0.91 | 1 | 1.08 | 1.14 |
| 0 day | Area% | 0.01 | 0.01 | / | 0.02 | 0.07 | 99.76 | 0.08 | 0.03 |
| | | | | | | | | | |
| 2-8°C 5 days | Area% | 0.05 | / | / | 0.01 | 0.05 | 99.82 | 0.06 | 0.02 |
| 2-8°C 10 days | Area% | 0.05 | / | / | 0.01 | 0.05 | 99.81 | 0.06 | 0.01 |
| 2-8°C 3.5 months | Area% | 0.06 | / | / | 0.02 | 0.06 | 99.40 | 0.06 | 0.03 |

| **Formulation 2 (Phosphoric Acid)** | RRT | 0.68 | 0.79 | 0.85 | 0.88 | 0.91 | 1 | 1.08 | 1.14 |
|---|---|---|---|---|---|---|---|---|---|
| | | | | | | | | | |
| 25°C 5 days | Area% | 0.09 | 0.01 | / | 0.02 | 0.07 | 99.73 | 0.06 | 0.02 |
| 25°C 10 days | Area% | 0.10 | 0.01 | / | 0.02 | 0.07 | 99.72 | 0.06 | 0.01 |
| 25°C 3.5 months | Area% | 0.15 | 0.02 | / | 0.02 | 0.06 | 99.64 | 0.06 | 0.03 |
| | | | | | | | | | |
| 40°C 5 days | Area% | 0.19 | 0.03 | / | 0.02 | 0.06 | 99.63 | 0.04 | 0.02 |
| 40°C 10 days | Area% | 0.23 | 0.03 | / | 0.02 | 0.07 | 99.59 | 0.05 | 0.02 |
| 40°C 3.5 months | Area% | 0.69 | 0.06 | 0.07 | 0.05 | 0.08 | 98.50 | 0.04 | 0.24 |

### HPLC-conditions for the determination of related substances of Anidulafungin:

### Method description:

| | |
|---|---|
| Column: | Phenomenex Gemini C18, 3 µm, 150 x 4.6 mm |
| Detection: | light absorption at 300 nm |
| Column temperature: | 30°C |
| Flow rate: | 1.5 mL/min |
| Injection volume: | 10 µL |
| Sample tray temp.: | 4°C |

### Mobile phases:

mobile phase A: Acetonitrile : Water : TFA (430:570:1)
mobile phase B: Acetonitrile : Water : TFA (800:200:1) (TFA: trifluoroacetic acid)

### Gradient program:

| **Time, min** | **Mobile phase A, %** | **Mobile phase B, %** |
|---|---|---|
| 0 | 100 | 0 |
| 28 | 100 | 0 |
| 40 | 0 | 100 |
| 50 | 0 | 100 |
| 51 | 100 | 0 |
| 60 | 100 | 0 |

### Example 2

Pharmaceutical formulations were prepared by the following method: Dissolve the pre-weighed amount of (S)-lactic acid in about 0.5% of total amount of water for injection (WFI) and pour into reactor, rinse weighing container with about 50 ml of WFI into reactor; stir for 10 min with a mechanical stirrer at a speed of 150-190 rpm. Adjust pH of solution to 4.3 with necessary volume of 1 M sodium hydroxide solution. After each addition of 1 M sodium hydroxide solution stir for not less than (NLT) 5 min with a mechanical stirrer at a speed of 150-190 rpm. Measure pH. If necessary, additional 1 M sodium hydroxide solution or 1 M hydrochloric acid solution is added to adjust pH to 4.3. Stir for NLT 5 min with a mechanical stirrer at a speed of 150-190 rpm.

Dissolve the pre-weighed amount of polysorbate 80 in about 75% of total amount of WFI, rinse weighing container with 100 ml of WFI into reactor; stir not less than 20 min until excipient completely dissolves with a mechanical stirrer at a speed of 150-190 rpm.

Add about 7% of total amount of WFI to the pre-weighed amount of the active pharmaceutical ingredient (API) anidulafungin in glass bottle, screw the cap, shake bottle well in order to promote wetting of API and transfer it into ultrasonic bath for homogenisation. Take not less than 3 cycles of homogenisation for 10 min. Slightly shake the bottle after each cycle, in order to rinse API particles from the walls of the bottle's upper part into suspension. After white, well-homogenized suspension is obtained, transfer it into intermediate product preparation reactor, stir with a mechanical stirrer at a speed of 150-190 rpm until substance dissolves completely.

Add the pre-weighed amounts of fructose and mannitol into reactor, stir until excipients completely dissolve with a mechanical stirrer at a speed of 150-190 rpm.

Add the necessary amount of WFI into reactor in order to reach total weight of intermediate product, stir NLT 5 min with a mechanical stirrer at a speed of 150-190 rpm.

If necessary, adjust pH to 4.3 by additional of 1 M hydrochloric acid solution or 1 M sodium hydroxide solution. After each addition of 1 M hydrochloric acid solution or 1 M sodium hydroxide solution, stir solution for NLT 5 min with a stirrer at a speed of 150-190 rpm.

The solution is filtered through sterile filters and lyophilized.

The anidulafungin powder had a D90 value of 339 µm based on volume distribution. 100% of the particles were below 750 µm. Particle sizes were analysed by laser diffraction with dry dispersion using a Malvern Mastersizer 3000 instrument. The size distribution was determined from the light scattering data using the theory of light scattering developed by Gustav Mie. This powder could not be dissolved completely in the polysorbate 80 solution even under vigorous stirring for 4 h. It remained an undissolved residue of about 5% which corresponds to particles with more than about 400 µm. About 9-10% of the particles were bigger than 350 µm. Only after the powder had been treated in an ultrasonic bath as described above complete dissolution could be achieved.

In a second experiment an aliquot of the anidulafungin powder was sieved through a 50 mesh sieve, corresponding to a mesh width of 297 µm to remove the bigger particles. The sieved powder could be dissolved easily in the polysorbate 80 solution with a mechanical stirrer.

## Claims

1. A process for making a freeze-dried pharmaceutical formulation comprising the steps of (i) preparing an aqueous solution comprising anidulafungin, a solubilizing agent, a stabilizing agent, a bulking agent, a buffer, and (ii) freeze drying the solution of step (i), wherein the anidulafungin is used in the form of a powder which does not contain particles having a size of more than 400 µm.

2. The process of claim 1, wherein the anidulafungin is used in the form of a powder which does not contain particles having a size of more than 350 µm.

3. The process of the claims 1 or 2 comprising the following order of steps:
(a) dissolving the buffer in water for injection and adjust the pH with sodium hydroxide to 3.0 -6.0;
(b) adding the solubilizing agent, the anidulafungin, the stabilizing agent, the bulking agent and the remaining water for injection, and stir the solution;
(c) sterile filtering of said solution;
(d) freeze-drying of said solution.

4. The process of any one of claim 1 to 3, wherein the freeze-dried pharmaceutical formulation comprises:
(a) anidulafungin;
(b) a solubilizing agent which is a pharmaceutically acceptable nonionic surfactant;
(c) a stabilizing agent which is a monosaccharide or disaccharide;
(d) a bulking agent which is mannitol;
(e) a buffer which is lactic acid or phosphoric acid;
(f) pH adjusting agent(s) which is sodium hydroxide or hydrochlorid acid.

5. The process of any one of claims 1 to 4, wherein said stabilizing agent is fructose.

6. The process of anyone of claims 1 to 5 wherein the solubilizing agent is a sorbitan polyoxyethylened derivative (polysorbate), preferably polysorbate 80 or polysorbate 20.

7. The process of any one of claim 1 to 6, wherein the pharmaceutical formulation comprises anidulafungin, mannitol, fructose, polysorbate, lactic acid or phosphoric acid, and a pH adjusting agent such as sodium hydroxide or hydrochloric acid.

8. The process of any one of claims 1 to 7, wherein the anidulafungin is used in the form of a dispersion.

9. A process for making a pharmaceutical formulation comprising reconstitution of a formulation as obtained by any one of the claims 1 to 8 in pharmaceutically acceptable liquid carrier, preferably in water for injection.

10. The process of claim 9, wherein the pharmaceutical formulation is suitable for parenteral administration.

11. The process of claim 9 or 10, wherein the pharmaceutical formulation is for use in the treatment of a fungal infection.

12. The process of any one of claims 9 to 11, wherein the pharmaceutical formulation comprises 10 mg/ml of anidulafungin, 10 mg/ml of fructose, 50 mg/ml of mannitol, 25 mg/ml of polysorbate 80, 7.5 mmol/L of lactic acid, and is adjusted to about pH 3.0 - 6.0, preferably 4.0 - 5.0 with sodium hydroxide or hydrochloric acid.

13. The process of any one of claims 9 to 12, wherein the pharmaceutical formulation comprises 10 mg/ml of anidulafungin, 10 mg/ml of fructose, 50 mg/ml of mannitol, 25 mg/ml of polysorbate 80, 7.5 mmol/L of phosphoric acid, and is adjusted to about pH 3.0 - 6.0, preferably 4.0 - 5.0 with sodium hydroxide or hydrochloric acid.

14. The process of any one of claims 1 to 13, wherein the pharmaceutical composition is essentially free of tartaric acid.

## Patentansprüche

1. Verfahren zur Herstellung einer gefriergetrockneten pharmazeutischen Formulierung, bei dem man (i) eine wässrige Lösung herstellt, die Anidulafungin, einen Lösungsvermittler, ein Stabilisierungsmittel, einen Füllstoff und einen Puffer enthält, und (ii) man die Lösung von Schritt (i) gefriertrocknet, wobei das Anidulafungin in der Form eines Pulvers verwendet wird, das keine Teilchen enthält, die eine Größe von mehr als 400 µm aufweisen.

2. Verfahren nach Anspruch 1, bei dem man das Anidulafungin in der Form eines Pulvers verwendet, das keine Teilchen enthält, die eine Größe von mehr als 350 µm aufweisen.

3. Verfahren nach den Ansprüchen 1 oder 2, bei dem man in der folgenden Reihenfolge
(a) den Puffer in Wasser zur Injektion löst und den pH mit Natriumhydroxid auf 3,0 bis 6,0 einstellt,
(b) man den Lösungsvermittler, das Anidulafungin, das Stabilisierungsmittel, den Füllstoff und das verbleibende Wasser zur Injektion hinzugibt und man die Lösung rührt,
(c) man die Lösung steril filtriert und
(d) man die Lösung gefriertrocknet.

4. Verfahren nach einem der Ansprüche 1 bis 3, bei dem die gefriergetrocknete pharmazeutische Formulierung
(a) Anidulafungin,
(b) einen Lösungsvermittler, der ein pharmazeutisch annehmbares nicht-ionisches Tensid ist,
(c) ein Stabilisierungsmittel, das ein Monosaccharid oder Disaccharid ist,
(d) einen Füllstoff, der Mannitol ist,
(e) einen Puffer, der Milchsäure oder Phosphorsäure ist, und
(f) Mittel zur pH-Einstellung, das/die Natriumhydroxid oder Salzsäure ist/sind,
enthält.

5. Verfahren nach einem der Ansprüche 1 bis 4, bei dem das Stabilisierungsmittel Fructose ist.

6. Verfahren nach einem der Ansprüche 1 bis 5, bei dem das Stabilisierungsmittel ein polyoxyethyleniertes Sorbitanderivat (Polysorbat), bevorzugt Polysorbat 80 oder Polysorbat 20 ist.

7. Verfahren nach einem der Ansprüche 1 bis 6, bei dem die pharmazeutische Formulierung Anidulafungin, Mannitol, Fructose, Polysorbat, Milchsäure oder Phosphorsäure, und ein Mittel zur pH-Einstellung, wie z.B. Natriumhydroxid oder Salzsäure, enthält.

8. Verfahren nach einem der Ansprüche 1 bis 7, bei dem Anidulafungin in der Form einer Dispersion verwendet wird.

9. Verfahren zur Herstellung einer pharmazeutischen Formulierung, bei dem man eine Formulierung, wie sie nach einem der Ansprüche 1 bis 8 erhalten wurde, in einem pharmazeutisch annehmbaren flüssigen Träger, bevorzugt in Wasser zur Injektion, rekonstituiert.

10. Verfahren nach Anspruch 9, bei dem die pharmazeutische Formulierung für die parenterale Verabreichung geeignet ist.

11. Verfahren nach Anspruch 9 oder 10, bei dem die pharmazeutische Formulierung zur Verwendung bei der Behandlung einer Pilzinfektion vorgesehen ist.

12. Verfahren nach einem der Ansprüche 9 bis 11, bei dem die pharmazeutische Formulierung 10 mg/ml Anidulafungin, 10 mg/ml Fructose, 50 mg/ml Mannitol, 25 mg/ml Polysorbat 80 und 7,5 mmol/l Milchsäure enthält und mit Natriumhydroxid oder Salzsäure auf einen pH von etwa 3,0 bis 6,0, bevorzugt 4,0 bis 5,0, eingestellt wird.

13. Verfahren nach einem der Ansprüche 9 bis 12, bei dem die pharmazeutische Formulierung 10 mg/ml Anidulafungin, 10 mg/ml Fructose, 50 mg/ml Mannitol, 25 mg/ml Polysorbat 80 und 7,5 mmol/l Phosphorsäure enthält und mit Natriumhydroxid oder Salzsäure auf einen pH von etwa 3,0 bis 6,0, bevorzugt 4,0 bis 5,0, eingestellt wird.

14. Verfahren nach einem der Ansprüche 1 bis 13, bei dem die pharmazeutische Formulierung im Wesentlichen frei von Weinsäure ist.

## Revendications

1. Procédé pour la production d'une composition pharmaceutique lyophilisée, comprenant les étapes consistant à (i) préparer une solution aqueuse comprenant de l'anidulafungine, un agent de solubilisation, un agent stabilisant, un agent de charge, un tampon, et (ii) lyophiliser la solution de l'étape (i), dans lequel on utilise l'anidulafungine sous la forme d'une poudre qui ne contient pas de particules ayant une taille de plus de 400 µm.

2. Procédé selon la revendication 1, dans lequel on utilise l'anidulafungine sous la forme d'une poudre qui ne contient pas de particules ayant une taille de plus de 350 µm.

3. Procédé selon la revendication 1 ou 2, comprenant l'ordre suivant d'étapes consistant à :
(a) dissoudre le tampon dans de l'eau pour injection et ajuster le pH à 3,0 - 6,0 à l'aide d'hydroxyde de sodium ;
(b) ajouter l'agent de solubilisation, l'anidulafungine, l'agent stabilisant, l'agent de charge et le reste de l'eau pour injection, et agiter la solution ;
(c) stériliser par filtration ladite solution ;
(d) lyophiliser ladite solution.

4. Procédé selon l'une quelconque des revendications 1 à 3, dans lequel la composition pharmaceutique lyophilisée comprend :
(a) de l'anidulafungine ;
(b) un agent de solubilisation qui est un tensioactif non ionique pharmaceutiquement acceptable ;
(c) un agent stabilisant qui est un monosaccharide ou disaccharide ;
(d) un agent de charge qui est le mannitol ;
(e) un tampon qui est l'acide lactique ou l'acide phosphorique ;
(f) un(des) agent(s) d'ajustement du pH qui est(sont) l'hydroxyde de sodium ou l'acide chlorhydrique.

5. Procédé selon l'une quelconque des revendications 1 à 4, dans lequel ledit agent stabilisant est le fructose.

6. Procédé selon l'une quelconque des revendications 1 à 5, dans lequel l'agent de solubilisation est un dérivé polyéthoxylé de sorbitane (polysorbate), de préférence le polysorbate 80 ou le polysorbate 20.

7. Procédé selon l'une quelconque des revendications 1 à 6, dans lequel la composition pharmaceutique comprend de l'anidulafungine, du mannitol, du fructose, du polysorbate, de l'acide lactique ou de l'acide phosphorique, et un agent d'ajustement du pH tel que l'hydroxyde de sodium ou l'acide chlorhydrique.

8. Procédé selon l'une quelconque des revendications 1 à 7, dans lequel on utilise l'anidulafungine sous la forme d'une dispersion.

9. Procédé pour la production d'une composition pharmaceutique, comprenant la reconstitution d'une composition telle qu'obtenue par l'une quelconque des revendications 1 à 8 dans un véhicule liquide pharmaceutiquement acceptable, de préférence dans de l'eau pour injection.

10. Procédé selon la revendication 9, dans lequel la composition pharmaceutique est appropriée à l'administration parentérale.

11. Procédé selon la revendication 9 ou 10, dans lequel la composition pharmaceutique est destinée à l'utilisation dans le traitement d'une infection fongique.

12. Procédé selon l'une quelconque des revendications 9 à 11, dans lequel la composition pharmaceutique comprend 10 mg/ml d'anidulafungine, 10 mg/ml de fructose, 50 mg/ml de mannitol, 25 mg/ml de polysorbate 80, 7,5 mmoles/L d'acide lactique, et est ajustée à environ pH 3,0 - 6,0, de préférence 4,0 - 5,0 avec de l'hydroxyde de sodium ou de l'acide chlorhydrique.

13. Procédé selon l'une quelconque des revendications 9 à 12, dans lequel la composition pharmaceutique comprend 10 mg/ml d'anidulafungine, 10 mg/ml de fructose, 50 mg/ml de mannitol, 25 mg/ml de polysorbate 80, 7,5 mmoles/L d'acide phosphorique, et est ajustée à environ pH 3,0 - 6,0, de préférence 4,0 - 5,0 avec de l'hydroxyde de sodium ou de l'acide chlorhydrique.

14. Procédé selon l'une quelconque des revendications 1 à 13, dans lequel la composition pharmaceutique est essentiellement exempte d'acide tartrique.
